Europäisches Patentamt

European Patent Office

Office européen des brevets

(11) Veröffentlichungsnummer: **0 258 258**
**B1**

(12) **EUROPÄISCHE PATENTSCHRIFT**

(45) Veröffentlichungstag der Patentschrift:
**29.03.89**

(21) Anmeldenummer: **86904132.7**

(22) Anmeldetag: **19.06.86**

(86) Internationale Anmeldenummer:
**PCT/EP 86/00359**

(87) Internationale Veröffentlichungsnummer:
**WO 86/07547 (31.12.86 Gazette 86/28)**

(51) Int. Cl.⁴: **B 01 J 2/10,** F 26 B 5/04,
A 61 K 9/16, B 01 J 2/16,
B 29 B 13/06, F 26 B 7/00

(54) **VERFAHREN UND VORRICHTUNG ZUR BEHANDLUNG VON PROZESSGUT, SOWIE MIT HILFE DES VERFAHRENS UND/ODER DER VORRICHTUNG HERGESTELLTES REAKTIVPRODUKT.**

(30) Priorität: **21.06.85 CH 2640/85**
**03.10.85 CH 4267/85**

(43) Veröffentlichungstag der Anmeldung:
**09.03.88 Patentblatt 88/10**

(45) Bekanntmachung des Hinweises auf die Patenterteilung:
**29.03.89 Patentblatt 89/13**

(84) Benannte Vertragsstaaten:
**AT BE CH DE FR GB IT LI LU NL SE**

(56) Entgegenhaltungen:
**AT-B-372 299**
**AU-D-1 264 466**
**DE-B-1 060 093**
**FR-A-2 224 269**
**FR-A-2 406 347**
**FR-A-2 422 915**
**GB-A-2 031 570**
**US-A-3 921 309**

(73) Patentinhaber: **Gergely, Gerhard, Dr., Gartengasse 8, A-1050 Wien (AT)**

(72) Erfinder: **GERGELY, Thomas, Gartengasse 8, A-1050 Wien (AT)**
Erfinder: **GERGELY, Irmgard, Gartengasse 8, A-1050 Wien (AT)**
Erfinder: **GERGELY, Gerhard, Gartengasse 8, A-1050 Wien (AT)**

(74) Vertreter: **Büchel, Kurt F., Dr., Patentanwalt Dr. Kurt F. Büchel Bergstrasse 297, FL- 9495 Triesen (LI)**

**Beschreibung**

Die Erfindung betrifft zunächst ein Verfahren nach dem Oberbegriff des Anspruches 1. Die Erfindung betrifft des weiteren eine Vorrichtung nach dem Oberbegriff des Anspruches 10, sowie ein Reaktivprodukt, das mit Hilfe des Verfahrens und/oder der Vorrichtung hergestellt ist.

Insbesondere in der pharmazeutischen Industrie ist es immer wieder erforderlich, Pulver und Granulate zu mischen, zu erwärmen, zu befeuchten und zu trocknen, od.dgl.. Diese Behandlungsschritte erfordern je nach Art und Menge der Rohstoffe oft verschiedenartiges Vorgehen. Manche Rohstoffe dürfen nur schonend bewegt und/oder erhitzt werden, andere neigen beim Befeuchten und Trocknen zur Bildung von Klumpen und/oder einer die vollständige Trocknung behindernden Haut; häufig ist auch die gleichmässige Verteilung von Lösungs- oder Benetzungsmitteln, verhältnismässig kleiner Mengen von Hilfs- oder Wirkstoffen, etc. schwierig oder zumindest langwierig.

Es wurden daher schon die verschiedensten Methoden und Vorrichtungen vorgeschlagen, die diese Probleme lösen sollten, was aber bisher immer noch in vielen Fällen nur unbefriedigend möglich ist. So erfordert die Granulation von Pulvern immer noch eine mehrstufige, die Trocknung insbesondere grösserer Chargen eine oft stundenlang dauernde Behandlung. Dies gilt auch für Trocknungsvorgänge, bei denen Heissluft durch oder über die Schüttung eines befeuchteten Gutes gesaugt und/oder die Feuchtigkeit durch das Anlegen von Vakuum entfernt werden soll. Dabei kommt es oft dazu, dass Gemischpartikel in der Schüttung zwar oberflächlich erwärmt werden und antrocknen, wobei aber z. B. vorhandene Bindemittel - wie oben erwähnt - eine Haut bilden, die eine weitere Trocknung erschwert oder gar verunmöglicht. Durch das Anlegen von Vakuum kühlt die Mischung andererseits stark ab, weil das verdampfende Lösungsmittel dem Gut die Verdampfungswärme entzieht, wodurch die Trocknung trotz angelegtem Vakuum zumindest verlangsamt wird.

Gemäss Stand der Technik wird z. B. das Prozessgut mit flüssigem Behandlungsmittel vermischt und dann gegebenenfalls von diesem bzw. von dessen verdampfbaren Anteilen durch Erwärmen und/oder Druckverminderung befreit. Die exakte Dosierung und gleichmässige Verteilung des Behandlungsmittels kann - wie bereits erwähnt, besonders bei verhältnismässig geringen Behandlungsmittelanteilen, z. B. solchen, die zum Anteigen bzw. zur Bildung einer fliessfähigen Dispersion nicht ausreichen, bei grosser Oberfläche des Schüttgutes und/oder bei grossen Dichteunterschieden - zu Problemen führen; auch die Entfernung der verdampfbaren Anteile des Behandlungsmittels ist nicht immer einfach, insbesondere, wenn sich das Prozessgut schlecht bzw. nur mit hohem Energie- oder grossem Verfahrensaufwand wieder abtrennen lässt. Bei polaren Lösungsmitteln einschliesslich von Wasser als Behandlungsmittel sind diese Probleme wegen der grossen chemischen und physikalischen Reaktionsfähigkeit dieser Mittel meist besonders ausgeprägt, wobei die Grenzen zwischen "chemischen" und "physikalischen" Bindungen bzw. Bindungsenergien bei Systemen mit verhältnismässig grossen Oberflächen oft verschwimmen. Man ist daher häufig gezwungen, auf die Verwendung eines an sich brauchbaren und vorteilhaften, weil billigen und ökologisch problemlosen Behandlungsmittels zu verzichten und dieses entweder durch ein anderes Mittel zu ersetzen oder zu "verdünnen"; hinzu kommt die zunehmende Bedeutung energiesparender und umweltschonender Massnahmen bei der Verarbeitung feinteiliger Substanzen in praktisch allen Bereichen der Technik von der Nahrungsmittel- und Waschmittelverarbeitung über die Arzneimittelverarbeitung bis zur Düngemittel- und Baustofftechnik.

Die theoretischen Vorgänge bei der Be- und Verarbeitung von Pulvern hatte man bisher noch immer nicht im Griff, sondern war im wesentlichen althergebrachten Verfahren verhaftet. Z.B. wird bei der Herstellung von Heilmitteln noch immer Stärke als Bindemittel verwendet, wobei für jede Charge eine bestimmte Menge Stärke mit erheblichen Mengen Wasser hydrolisiert wird, welches anschliessend in langwierigen Trocknungsvorgängen wieder entfernt werden muss; es wird so das Agglomerieren oder Granulieren der Masse erzielt, die anschliessend auf die gewünschte Korngrösse zerkleinert wird. Aus diesem Grund ist auch die Automation dieser Verfahrensschritte bisher nicht weit gediehen.

Wenn man jedoch von einem in Einzelschritte aufgelösten Verfahren ausgeht und dafür Sorge trägt, dass einerseits genügend Wärme und/oder anderseits das Behandlungsmittel in der gewünschten Konzentration in diesen Stufen eingebracht wird, dann gelangt man zu einer optimalen und vor allem auch reproduzierbaren Lösung der eingangs genannten Aufgaben.

Will man ein Kohlehydrat, wie beispielsweise Lactose oder Zucker, mit einem Wirkstoff granulieren, dann wird die Pulvermischung mit Wasser behandelt, wobei sich einige Teile auflösen und nach dem Trocknen grobkörnige Strukturen zeigen. Eine vollständige Uniformität der Zusammensetzung und der Korngrösse ist keineswegs gewährleistet.

Ein spezielles Beispiel für die besonderen Aufgabenstellungen der Behandlung von schüttfähigem festem Prozessgut ist das Spezialgebiet der Agglomerierung von pulverförmigen Mischungen mit oder ohne chemische Umsetzung im Grenzflächenbereich, wie insbesondere die Herstellung bzw. Verarbeitung der in Gegenwart von Wasser unter Entwicklung von $CO_2$ reaktionsfähigen Zubereitungen, im folgenden kurz "Reaktivprodukte" genannt.

Die Herstellung solcher Reaktivprodukte ist aber nur eines von vielen Beispielen für die Probleme der Behandlung von feinteiligem Prozessgut; ein anderes Beispiel ist die Granulierung von sprühgetrockneten oder durch Feinmahlung erhaltenen Substanzen oder die Durchführung von chemischen Oberflächenreaktionen.

Die Erfindung hat sich nun zur Aufgabe gestellt, ein Verfahren und eine Vorrichtung zu schaffen, mittels derer es in verhältnismässig kurzer Zeit möglich ist, eine Schüttung von Pulver oder Granulat sehr gleichmässig zu behandeln und gegebenenfalls zu trocknen, auch wenn es sich um diesbezüglich empfindliche, bzw. störungsanfällige Substanzen handelt.

Das Verfahren soll eine genaue Kontrolle des Behandlungsablaufes sowie das rasche und einfache Erreichen einer nahezu beliebig gleichmässigen Behandlung bzw. Verteilung von Behandlungsmittel auch dann ermöglichen, wenn dessen Anteil im Verhältnis zum Prozessgut sehr klein oder die Tendenz zu unerwünschten oder unerwünscht starken Wechselwirkungen zwischen Behandlungsmittel und Prozessgut besonders gross bzw. die Entfernung des Behandlungsmittels normalerweise sehr energieverbrauchend ist.

Diese Aufgaben gelingen überraschend mit einem Verfahren nach dem Kennzeichen des Anspruches 1. Bevorzugte Ausführungsformen dieses Verfahrens sind in den Kennzeichen der Ansprüche 2 bis 9 beschrieben.

Beide erfindungsgemässen Massnahmen bringen schon für sich allein mehr Wärme ein und führen zu einer gleichmässigeren Behandlung als es ohne eine der Massnahmen der Fall wäre. Noch besser ist natürlich ihre gleichzeitige Anwendung.

Bei der erfindungsgemässen Behandlung wird z. B. für die Granulierung eines Brausegemisches die Heissluft mit Dampf beladen, der dann kondensiert; anschliessend kann man den Dampf wegschalten, mit Heissluft die gebildete Kohlensäure und Feuchtigkeit abtransportieren und sodann unter Vakuum fertigtrocknen. Vorzugsweise wird der Heissluftstrom deshalb mit 1,5 bar eingedrückt, weil beim Ansaugen, um den Widerstand des Wärmetauschers zu verhindern, zu hohe Vakua nötig wären. Dies aber würde das Kondensieren des Dampfes verhindern oder zumindest verringern.

In der prioritätsälteren, aber nicht vorveröffentlichten EP-A-151 782 wird die einmalige Behandlung eines Schüttgutes in einer Vakuumtrommel mit einem - gegebenenfalls mit Dampf beladenen - Heissluftstrom geoffenbart, der durch die Schüttung hindurchgeführt wird. Dabei treten mehrere Nachteile auf:

- beim Durchleiten durch die Mischung können sich die Düsen leicht mit noch feuchtem, feinkörnigem Gut verstopfen;
- um die Behandlung in einem Durchgang zu erzielen, muss die Temperatur und/oder Dampfkonzentration relativ hoch sein; das aber führt zu lokalen Überhitzungen und/oder ungleichmässiger Kondensation.

In gleicher Weise nachteilig ist der in der DE-B-1 060 093 beschriebene einmalige Vorgang, bei dem übrigens nicht notwendigerweise erwärmt, sondern zwecks Granulierung eines Pulvers ein dampfförmiges Lösungsmittel einmalig eingesaugt und nach Kondensation durch Vakuum wieder entfernt wird.

Nun wurde in der GB-A-2 031 570 ein Trocknungsverfahren beschrieben, bei dem flüssige oder pastenförmige Produkte intermittierend einem Unterdruck und dann wieder einem erwärmten, trockenen Gasstrom ausgesetzt werden; dabei treten jedoch sogar bei verhältnismässig wenig Feuchtigkeit enthaltenden Produkten, wie Hefe oder Zwiebeln, enorm lange Trocknungszeiten auf, die nur im Vergleich mit den noch viel längeren Gefriertrocknungszeiten vorteilhaft erscheinen. Dies hängt damit zusammen, dass der Luftstrom im Kreis geführt und über ein Absorberbett von Lösungsmitteldampf befreit wird, wobei es zu einem beträchtlichen Druckabfall kommt. Daher dauert einerseits die Ansaugphase zu lang; andererseits bringt der Luftstrom nur wenig Wärme mit, um das durch das Anlegen von Vakuum zu stark abgekühlte Gut wieder aufzuwärmen.

Ein für die Luftvorwärmung üblicher Wärmetauscher hat einen bestimmten Strömungswiderstand. Wird dieser nun in herkömmlicher Weise durch einen Saugvorgang überwunden, entsteht im Kessel ein verhältnismässig hohes Vakuum und damit eine starke Abkühlung des Gutes, bzw. ein hoher Wärmeverlust. Durch komprimierte Luft wird hingegen der Reibungsverlust im Wärmetauscher durch einen Kompressor kompensiert.

Dadurch, dass bisher die Heissluft im wesentlichen nur durch oder über das Gut gesaugt wurde, ergab sich beispielsweise durch die Verdampfung der zu trocknenden Feuchtigkeit eine Temperaturerniedrigung, die erst langsam durch nachströmende Heissluft wettgemacht werden konnte; ein Grossteil der Wärmeenergie ging verloren. Achtete man aber auf einen geringeren Wärmeverlust durch vorgängiges Aufheizen der Mischung, ergaben sich wieder die vorher erwähnten Probleme, die erst mit dem erfindungsgemässen Verfahren gelöst werden konnten, bzw. die gar nicht erst auftreten, wenn die erwähnten Nachteile vermieden werden.

Die üblichen Behandlungsvorgänge zwischen einem z. B. pulverförmigen Prozessgut und einer Flüssigkeit, z. B. einem Lösungsmittel, werden in Einzelschritte zerlegt, die besser kontrollier- und steuerbar, sowie gegebenenfalls in Richtung einer Automation sogar programmierbar sind. Dies schien dem Fachmann im Zusammenhang mit solchen Behandlungsvorgängen bisher nicht möglich, da viele Flüssigkeiten, wie z. B. Bindemittellösungen oder sogar Wasser allein noch immer viel zu viskos sind, um im Mikrobereich von Prozessgutpartikeln einer Grössenordnung zwischen 10 und 100 micron eine gleichmässige Verteilung erzielen zu lassen. Man musste also bisher immer einen sehr grossen Überschuss an Lösungsmittel zugeben, um eine sichere Benetzung aller Teilchen zu erzielen.

Mischt man hingegen Bindemittel, Füllstoff und Wirkstoff in Pulverform und behandelt anschliessend stufenweise mit ganz geringen Mengen des Lösungsmittels, dessen Viskosität drastisch absinkt, wenn es in der Dampfphase und durch Heissluft verdünnt vorliegt, dann werden, insbesondere im Zusammenhang mit den weiteren erfindungsgemäss bevorzugten Massnahmen, die bisher unüberwindlich erscheinenden Probleme gelöst.

Wasserdampf allein führt dabei noch nicht in allen Fällen zu brauchbaren Ergebnissen, weil die Kondensation des Wasserdampfes an der Stelle seines Eintrittes an einer kühleren Fläche, also an den zu granulierenden Partikeln, viel zu rasch erfolgt und zur Klumpenbildung führt. Enthält aber z. B. 10 Liter Luft von 120 Grad C nur 100 ml Wasser, dann erfolgt die Kondensation wesentlich langsamer, bevor das Wasser flüssig wird. Die langsame Kondensation einer Flüssigkeit aus der Dampfphase in feinsten Tröpfchen (Nebelbildung) ist auch bei vielen anderen Vorgängen ungleich wirkungsvoller und energetisch sparsamer als die Behandlung mit Flüssigkeit.

Die für eine reproduzier- und programmierbare Vorgangsweise erforderlichen Parameter sind die Temperatur des Prozessgutes und der zugeführten Luft, der Anteil des dampfförmigen Reaktionspartners, bzw. Behandlungsmittels in der Verdünnungsphase, und der gegebenenfalls gewählte Unterdruck. Man kann mit dieser Vorgangsweise die Benetzung der Prozessgut-Teilchen in mehrere Stufen zerlegen und den Aufbau eines Granulates, d.h. die Kornvergrösserung exakt einstellen, verfolgen und den Vorgang rechtzeitig abbrechen.

Behandelt man z. B. eine Mischung von 80 Teilen Lactose, 15 Teilen eines pharmazeutischen Wirkstoffes und 5 Teilen eines Bindemittels etwa 10 mal hintereinander mit jeweils nur 3 Teilen Wasser in ca. 15 Teilen einströmender Heissluft und trocknet nach jeder Behandlung durch Anlegen von Vakuum, dann erreicht man die erwähnte wünschenswerte Kontrolle des Verfahrens und braucht in der programmierten Schaltung der später noch beschriebenen Vorrichtung jeweils nur drei Steuerteile zu schalten, nämlich das Einströmventil für das voreingestellte Dampf-Luft-Gemisch, das Ventil zur Vakuumpumpe zwecks Wechsel von einem niedrigen Konstantvakuum für die Kondensation zu einem Vollvakuum für das Trocknen, und schliesslich die Steuerung der Rührgeschwindigkeit, die während der Kondensation hoch, während der Trocknung aber niedrig sein soll.

Die in die Trommel gedrückte und/oder mit Dampf, der eine weit höhere spezifische Wärme als die Luft besitzt, beladene Heissluft gibt ihre Wärme an das Gut ab, was zu dem gewünschten Behandlungseffekt, z. B. zu einer Verdampfung der Feuchtigkeit an der jeweiligen Kornoberfläche führt. Da vorher Unterdruck in der Schüttung geherrscht hat, dringt die Heissluft bis in alle Bereiche der Schüttung vor.

Dieses Einströmen ist wichtig und erfindungsgemäss dem üblichen Durchsaugen durch das Gut hoch überlegen. Selbst eine sekundäre Luftumwälzung im Hohlraum über der Schüttung im Vakkum-Mischkessel gibt eine Verwirbelung nur in eben diesem Luftraum, bzw. wirbelt sie sogar womöglich Staub auf.

Natürlich muss man bei einfachen Trocknungsvorgängen dafür sorgen, den Dampfgehalt der Luft zu optimieren. Mehr Dampf gibt zwar mehr Wärme an das Gut ab, verhindert aber aus Gleichgewichtsgründen, dass mehr Feuchtigkeit verdampft. Dies ist aber eine für den Fachmann in Abhängigkeit von dem zu behandelnden Gut leicht zu ermittelnde Grösse, bzw. durchzuführende Massnahme.

In der anschliessenden Phase, in der Vakuum angelegt wird, wird diese Feuchtigkeit abgesaugt. Dadurch, dass die Vakuumphase in aller Regel nur einen Bruchteil der Heissluft-Druckphase ausmacht, geht nur die minimale, absolut notwendige Wärmemenge verloren. Der Wärmeverlust kann noch weiter verringert werden, wenn die Heissluftzufuhr während wenigstens eines Teils der Absaugphase unterbrochen wird.

Sollte es in dieser Phase zur Ausbildung einer Haut an der Kornoberfläche kommen, dann erhält die Feucitigkeit im Inneren der Körner während der anschliessenden Heissluft-Druckphase wieder Gelegenheit, nach aussen zu diffundieren ("Migration"), die dort gebildete Haut zu lösen und in die Heissluft zu verdampfen.

Die Temperatur der Heissluft und die Zykluszeiten können ganz leicht auf das jeweils zu behandelnde Produkt eingestellt werden. Mit dem erfindungsgemässen Verfahren gelingt es, auch 100 oder mehr kg betragende Schüttungen feiner Pulver, wie z. B. Milchzucker, Staubzucker, Paracetamol, Naproxen etc., die beispielsweise 10 bis 20 % Feuchtigkeit enthalten, innerhalb von 30 bis 60, oft sogar nur von 20 Minuten zu trocknen. Die Steuerung der Zykluszeit und der Abbruch des Verfahrens können z. B. nach einer laufenden Feuchtemessung erfolgen.

Ist die Druckdifferenz zwischen dem Heisslufteintritt und der Absaugung in der Vakuumphase zu gross, dann ergeben sich - wenn auch in geringerem Ausmass - die oben erwähnten Nachteile des Standes der Technik wenigstens zum Teil.

Trotz der erwähnten Durchdringung der Schüttung mit Heissluft bzw. Vakuum hat es sich in vielen Fällen zur Vermeidung von Agglomerationen als zweckmässig erwiesen, wenigstens von Zeit zu Zeit die Schüttung durch eine Trommeldrehung und/oder die Bewegung eines Rührers zu bewegen. Eine zu intensive Bewegung sollte allerdings vermieden werden, weil dabei gebildetes oder in Bildung befindliches Granulat zerstört und besonders für die Vakuumpumpe unerwünschter Staub gebildet werden kann.

Zur Durchführung des erfindungsgemässen Verfahrens kann das teilchenförmige, z. B. Korngrössen von 0,01 bis 1,5 mm oder mehr aufweisende Prozessgut, gegebenenfalls als Vormischung der Komponenten oder in Form der einzelnen Komponenten, in den Prozessraum, z. B. eine Vakuumkammer mit mechanischen und/oder pneumatischen Einrichtungen zur Bewegung des Prozessgutes, eingeführt werden.

Geeignete Bewegungseinrichtungen sind solche, die auf das Gut höchstens geringe Scherkräfte ausüben, vorzugsweise solche, die eine Bewegung des Gutes entgegen der Schwerkraft bewirken, wie insbesondere die zwei- oder dreidimensional schwingenden Mischeinrichtungen. Ebenfalls bevorzugt verwendet werden Mischeinrichtungen, die sowohl eine mechanische als auch eine pneumatische Bewegung des Prozessgutes auslösen, z. B. hohle Mischarme, durch deren Öffnungen Heissgas eingeblasen werden kann.

Die Bezeichnung "heisses Gas" bzw. "Heissgas" bedeutet im Rahmen der Erfindung ein verhältnismässig, d.h. im Vergleich zum Behandlungsmitteldampf ideales Gas oder Gasgemisch, wie Stickstoff, Luft, Edelgas oder Kohlendioxid, soweit keine unerwünschten Reaktionen dadurch verursacht werden.

Die Temperatur des heissen Gases muss beim erfindungsgemässen Verfahren höher sein, als die Verdampfungs- bzw. Siedetemperatur des Behandlungsmittels bei Normaldruck von etwa 1 bar, typisch um mindestens 10 Grad C und vorzugsweise um mindestens 20 Grad C höher. Die Obergrenze der Temperatur des heissen Gases ist nicht besonders kritisch, wird jedoch aus Gründen der Wärmeempfindlichkeit des Prozessgutes sowie zur Vermeidung einer unnötigen thermischen Belastung der verwendeten Anlagen und aus Gründen der Minimierung des Energieverbrauchs nicht ohne besonderen Anlass wesentlich über die genannten Minimalwerte hinaus erhöht.

Für das erfindungsgemässe Verfahren geeignete Behandlungsmittel müssen bei Normaldruck im heissen Gas praktisch rückstandsfrei verdampfen (bzw. sublimieren) und bei Normaldruck und Temperaturen von typisch zwischen Null und 200 Grad C, vorzugsweise zwischen Raumtemperatur und 100 Grad C, kondensieren.

Bei Normaltemperatur flüssige und insbesondere polare Behandlungsmittel werden für das erfindungsgemässe Verfahren meist bevorzugt. Wasser ist bevorzugt, doch sind auch organische Lösungsmittel nicht ausgeschlossen. Ein für das erfindungsgemässe Verfahren geeignetes Behandlungsmittel muss befähigt sein, auf dem gegebenenfalls warmen Prozessgut in Form feinster Teilchen zu kondensieren und sich durch Verminderung des im Prozessraum herrschenden Druckes aus dem gegebenenfalls erwärmten Prozessgut leicht entfernen lassen.

Bei Verwendung von Wasser als Behandlungsmittel hat das heisse Gas, z. B. Luft, eine Temperatur von über 100 Grad C, vorzugsweise mindestens 110 - 150 Grad C, wobei die Kondensation auf dem typisch etwa 50 Grad warmen Prozessgut bei 200 - 900 mbar und das Verdampfen bei annähernd gleicher Prozessguttemperatur und beispielsweise bei 10 - 30 mbar erfolgt.

Die Konzentration des Behandlungsmitteldampfes im Heissgas kann in weiten Grenzen, z. B. von 0,1 bis 50 Vol-% oder mehr, verändert werden; die Verwendung von Mischungen mit höheren Behandlungsmitteldampfanteilen ist möglich, aber meist nicht bevorzugt; typische Heissluft/Wasserdampf-Mischungen enthalten z. B. pro Volumen Wasserdampf 1 bis 5, vorzugsweise 2 bis 4, Volumina Heissluft. Vor und/oder nach der Behandlung des Prozessgutes mit dem Behandlungsmittel bzw. Wasser kann Heissgas oder gewünschtenfalls auch kaltes Trägergas durch das Prozessgut geführt werden, um einen gewünschten Erwärmungs- oder Kühlungseffekt zu erzielen.

Zur Erzeugung der Mischung aus Heissgas und Behandlungsmitteldampf (nachfolgend Gas/Dampf-Gemisch genannt) kann das Behandlungsmittel in den Wärmetauscher eindosiert werden, der auch zur Erwärmung des Gases dient.

Vorzugsweise wird das in der Prozesskammer vorgelegte Gut vor Einführung des Gas/Dampf-Gemisches vorgewärmt, z. B. auf eine Temperatur, die deutlich unter der Normaldruck-Siedetemperatur des Behandlungsmittels, z. B. um 30 bis 60 Grad tiefer, liegt.

Diese Vorwärmung des Prozessgutes kann mit Heissgas und/oder durch Beheizung der Wand der Prozesskammer von aussen (Heizmantel) erfolgen. Der Wärmeübergang zwischen Kammerwand und Prozessgut kann dabei direkt, d.h. durch Kontakt des Prozessgutes mit der beheizten Wand oder - soferne kein erheblich verminderter Gasdruck herrscht - indirekt über das in der Kammer befindliche Gas/Dampf-Gemisch erfolgen.

Um die Kondensation des Behandlungsmitteldampfes auf den das Prozessgut bildenden Feststoffteilchen in möglichst gleichmässiger, feinstzerteilter Form zu bewirken, wird das Gas/Dampf-Gemisch bei normalem oder schwach vermindertem Druck allgemein so auf das vorzugsweise bewegte und erwärmte Prozessgut gebracht, dass sich zwischen bzw. auf den Prozessgutteilchen keine zusammehhängende Flüssigphase bildet; für viele Anwendungszwecke wird vorzugsweise so gearbeitet, dass die Prozessgutteilchen nicht "nass" in dem Sinne werden, dass sich auf jedem Teilchen ein zusammenhängender Flüssigkeitsfilm bildet bzw. der Raum zwischen den Teilchen praktisch mit Flüssigkeit gefüllt ist. Für eine optimale Verteilung des Behandlungsmittels auf bzw. in den Prozessgutteilchen wird bevorzugt, dass das Behandlungsmittel in Form von feinsten, d.h. normalerweise schwebfähigen Tröpfchen ("Nebel") auf den Prozessgutteilchen kondensiert und praktisch etwa ebenso rasch von den Prozessgutteilchen absorbiert, d.h. aufgesogen wird, oder mit ihrer Oberfläche reagiert wie es auf diesen kondensiert. Die Wahl der Druck-, Temperatur- und Mengenwerte für die Kondensation und die Anpassung an ein gegebenes Prozessgut kann anhand von einfachen Versuchen optimiert werden.

Der im folgenden als Kondensationsphase bezeichnete Schritt kann nach der Einführung des Gas/Dampf-Gemisches beendet oder über diesen Zeitpunkt hinaus zur Verteilung des Kondensates im Gut und/oder zur Wechselwirkung zwischen den Komponenten des Gutes verlängert werden; die Dauer dieser aus der eigentlichen Kondensation und der gegebenenfalls anschliessenden Behandlungsmitteleinwirkung bestehenden Phase beträgt typisch einige Sekunden bis einige Minuten, z. B. 10 sec bis 10 min, selten länger.

Die Kondensationsphase wird dadurch beendet, dass man den Druck in der Prozesskammer soweit vermindert, dass der Siedepunkt des Behandlungsmittels bei der herrschenden Temperatur des Prozessgutes und dem verminderten Druck mindestens erreicht und vorzugsweise überschritten wird.

Mit dieser Druckverminderung, z. B. aus 10 - 50 mbar, beginnt die Behandlungsmittel-Entfernungsphase, die zu einer teilweisen und typisch mindestens 50 %-igen oder praktisch vollständigen Entfernung des Behandlungsmittels führen kann; auch diese Phase kann typisch in Sekunden bis Minuten, z. B. 30 sec bis 30 min, beendet sein.

Jeweils eine Kondensationsphase mit nachfolgender Entfernungsphase wird hier als "Behandlungszyklus" bezeichnet; wie oben angedeutet, wird die Behandlung nach der Erfindung mindestens mit zwei, vorzugsweise mit fünf oder mehr Zyklen, durchgeführt, bis ein bestimmter Endzustand, z. B. ein gewünschter Agglomerations- und/oder Reaktions- und/oder Diffusionsgrad des Prozessgutes erreicht ist.

Auch in der AT-A-372 299 wird - im Gegensatz zur vorliegenden Erfindung - ein Lösungsmittel eingesaugt, dabei aber eine definierte Reaktion his zu einem definierten Druckanstieg ablaufen gelassen und anschliessend mit Vakuum getrocknet. Im gegenständlichen Fall jedoch wird mit Dampf bei 600 - 900 mbar das Dampf/Luftgemisch mit einem Überdruck von 0,5 bar, d.i. 1500 mbar eingedrückt und kondensieren gelassen, immer unter Unterdruck, und anschliessend wird entweder das entstehende $CO_2$ oder $H_2O$ mit Heissluft bei gleichen 600 - 900 mbar abgesaugt oder mit Vakuum getrocknet und der ganze Vorgang zyklisch wiederholt, d.h.,

man lässt das freie Volumen im Reaktionskessel nicht durch das durch Reaktion gebildete $CO_2$ und Wasser auffüllen, sondern es wird gleichzeitig während des Eindrückens des Dampf/Luftgemisches abgesaugt. Der Vorteil davon ist, dass das Produkt auf Temperatur bleibt, da der exotherme Vorgang durch die zugeführte Heissluft mit Dampf kondensiert wird; durch die feine Verteilung des Nebels ist die Reaktion nicht so heftig, kann aber leicht mehrfach durchgeführt werden.

Erfindungsgemässe Reaktivprodukte bestehen z. B. aus einer normalerweise festen, etwa kristallinen Carbonsäure, wie Zitronensäure, Weinsäure, Apfelsäure, Fumarsäure, Adipinsäure, Ascorbinsäure, oder Mischungen solcher Säuren, mit feinteiligen Reaktionspartnern, wie z. B. Carbonaten oder Bicarbonaten von Alkali- oder Erdalkalimetallen, Erdalkali-, Zink- oder Eisenoxiden oder -hydroxiden, oder anderen Salzen von Säuren, die schwächer sind, als die verwendete Carbonsäure, z. B. Zinkglycerophosphat, Eisengluconat, Calciumlaktat, etc..

Man erzielt eine optimierte Grenzfläche zwischen den Säure- und den Carbonatteilchen, wenn praktisch jedes Säureteilchen von Carbonatteilchen umhüllt ist; dabei versteht sich, dass ein Agglomerat auch mehrere Säureteilchen enthalten kann, von denen aber jedes praktisch von Carbonatteilchen umhüllt ist, wie weiter unten noch erläutert wird.

Hierbei können zusätzliche Binde- und Füllmittel, wie Zucker, Mannit, Stärke oder Lactose, verwendet werden; vorzugsweise dient als Bindemittel zwischen den Säureteilchen und den Carbonatteilchen eine durch Anreaktion in situ gebildete Verbindung aus der Säure und dem Carbonat, z. B. Calciumcitrat, die etwa 1 bis 5 % des Gewichtes der sie bildenden Komponenten ausmacht.

Durch entsprechende Zykluswiederholung können ferner nach dem erfindungsgemässen Verfahren zusätzliche Komponenten, z. B. Farb- oder Wirkstoffe, die in Wasser bei Prozessguttemperaturen eine Löslichkeit von mindestens etwa 5 g/Liter haben, gleichmässig in einem teilchenförmigen Prozessgut verteilt werden.

Insbesondere gelingt es auf diese Weise, neue Reaktivprodukte für Instant- oder Brause-Zubereitungen, bzw. mit schwerlöslichen Carbonaten, wie $MgCO_3$, $CaCO_3$ oder Oxiden, wie z. B. Mg-Oxid, sowie toxikologisch zulässigen Fluorverbindungen, wie Dinatriumfluorophosphat, in der für solche Produkte absolut notwendigen, gleichmässigen Verteilung herzustellen, wobei die Carbonatteilchen reaktiv oder anlösend in besonders einfacher und kostensparender Weise auf den Säureteilchen fixiert, bzw. in deren Oberfläche eingebettet werden. Ein besonders enger Kontakt zwischen den schwerlöslichen Reaktionspartnern mit den Säurekristallen ist wichtig; er wird durch den Zusatz von pulverisierter Säure erleichtert. Für andere schwerlösliche Reaktionspartner als z. B. Erdalkalicarbonate ist es zweckmässig, anschliessend leichter lösliche Alkalicarbonate zu verankern, wenn man Brausemischungen erzielen will.

Es ist überraschend, dass ein Reaktivprodukt mit Calciumcarbonat als $CO_2$-Bildner und mit einem Gehalt an wasserlöslichen Fluorverbindungen, z. B. in Anteilen von 0,05 bis 10 % des Gewichtes, erhältlich und brauchbar ist; vielmehr wäre zu erwarten gewesen, dass die lösliche Fluorverbindung sich bei der Herstellung quantitativ mit dem Calciumcarbonat bzw. Calciumcitrat zum unlöslichen Calciumfluorid umsetzen würde, und es ist nicht auszuschliessen, dass die genaue Kontrollierbarkeit des bevorzugt zur Herstellung solcher fluorhaltiger Reaktivprodukte angewendeten erfindungsgemässen Verfahrens eine ausschlaggebende Rolle spielt.

Auch schwerlösliche Oxide, wie z. B. Magnesiumoxid oder Magnesiumoxidcarbonat, Eisenoxid, Zinkoxid, etc. und ähnliche lassen sich durch dieses Verfahren so reaktiv an der Oberfläche einer Säure verankern, indem sie partiell mit den durch kondensierenden Nebel benetzten organischen Carbonsäuren reagieren und beim Trocknen überaus gleichmässig an der organischen Säure festgehalten sind.

Dadurch kann beim Einbringen der organischen Säure mit den angeklebten Oxiden in Wasser, sei es als Instant-Produkt oder als reaktives Brauseprodukt, der beim Herstellungsverfahren durch Wasserdampf initiierte Vorgang der Umsetzung zu Magnesiumcitrat, Eisencitrat, Zinkcitrat etc. fortgeführt werden.

Eine zur Durchführung des erfindungsgemässen Verfahrens besonders geeignete Vorrichtung und die Struktur bestimmter Reaktivprodukte werden anhand der beiliegenden Zeichnungen beispielhaft näher erläutert; es zeigen:

Fig. 1   das Schema einer Vorrichtung zur Durchführung des erfindungsgemässen Verfahrens, und die

Fig. 2   die schematische Schnittdarstellung
und 3    eines erfindungsgemäss hergestellten Agglomerates.

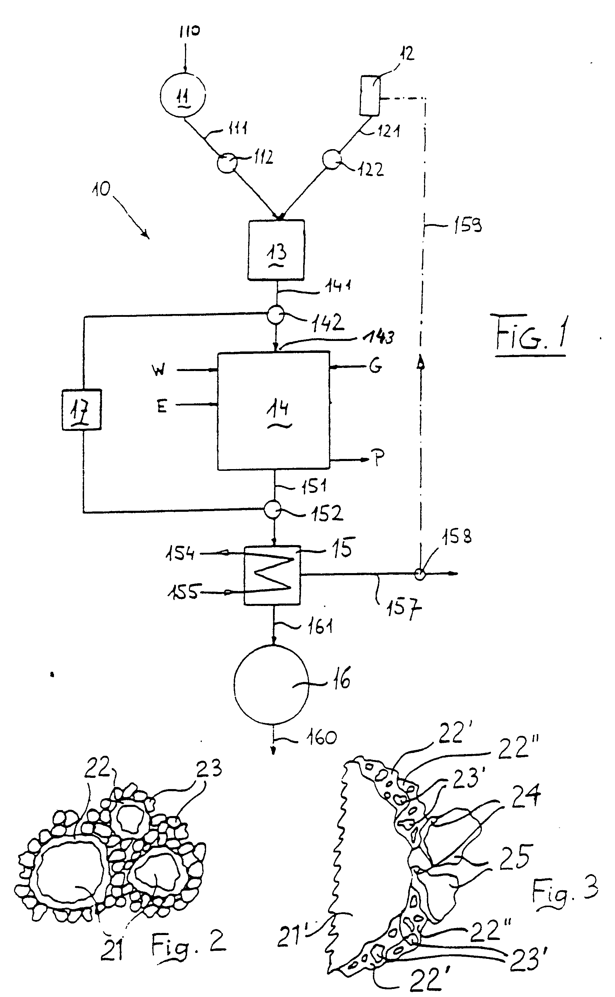

Im einzelnen zeigt Fig. 1 die schematisch dargestellte Vorrichtung 10; diese umfasst eine Pumpe bzw. einen Kompressor 11, die/der ein Gas, z. B. Luft, bei 110 ansaugt und durch die Leitung 111 mit dem fakultativen Ventil 112 in den Wärmetauscher 13 einspeist. Die Förderleistung beträgt beispielsweise 10 bis 100 Liter/Minute; der Druck, mit dem das Gas den Wärmetauscher verlässt, wird je nach den gewünschten Verfahrensparametern eingestellt z. B. auf 1,5 bar. Der Behälter 12 enthält das Behandlungsmittel und kann dieses über die Leitung 121 unter Dosierung durch das Ventil 122 in den Wärmetauscher 13 abgegeben, wo es - wenn gewünscht - zur Bildung der Mischung aus Heissgas und Behandlungsmitteldampf verdampft wird.

Der Wärmetauscher ist über die Leitung 141 und das Steuerventil 142 mit der Prozesskammer 14 verbunden, in die Prozessgut bzw. Prozessgutkomponenten G eingespeist, und aus der fertiges Produkt P abgezogen werden kann.

Die Prozesskammer 14 wird mit Wärme W, z. B. vermittels eines (nicht dargestellten) Heizmantels und mit Bewegungsenergie E, z. B. vermittels eines (nicht dargestellten) mechanisch und/oder pneumatisch wirkenden Rührers, zur Bewegung des Prozessgutes versorgt. Die Prozesskammer 14 ist ferner über die Leitung 151, das Steuerventil 152, einen fakultativen Kondensator 15 und die Leitung 161 mit einer Saug- bzw. Vakuumpumpe 16 verbunden, die das aus der Kammer 14 abgezogene Gas und gegebenenfalls das nicht im Kondensator 15 entfernte Behandlungsmittel durch die Leitung 160, gegebenenfalls über eine Reinigungsanlage, ins Freie abgibt oder (in nicht dargestellter Weise) zum Wärmetauscher 13 rezirkuliert. Die Leistung der Pumpe 16 ist vorzugsweise mindestens ebenso gross, wie die der Pumpe 11.

Der fakultative und bei Verwendung von Wasser als Behandlungsmittel nicht bevorzugte Kondensator 15 kann über die Kühlmittelleitungen 154, 155 in Betrieb gehalten werden, wobei das kondensierte Behandlungsmittel über die Leitung 157 ausgetragen bzw. gegebenenfalls über das Ventil 158 und die Rückführleitung 159 dem Behandlungsmittelbehälter 12 zugeführt wird.

Die Vorrichtung 10 hat für die Durchführung einer bevorzugten Ausführungsform des erfindungsgemässen Verfahrens eine Programmsteuerung 17, die im einfachsten Fall lediglich den Öffnungs/Schliess-Vorgang der Ventile 142, 152 sowie die Zeitpunkte der Ventilbetätigung steuert.

Vorausgesetzt, dass alle Teile der Vorrichtung 10 in Betrieb sind, kann die Kondensationsphase durch Öffnen des Ventils 142 bei ganz oder teilweise geschlossenem Ventil 152 eingeleitet und durch Schliessen des Ventils 142 und Öffnen des Ventils 152 beendet, d.h. die Verdampfungsphase eingeleitet werden.

Fig. 2 zeigt in schematischer Schnittdarstellung ein stereospezifisches Agglomerat, das eine Mehrzahl von Kernen 21 besitzt; die Kerne stellen z. B. die verhältnismässig grobteiligen Säurepartikel eines Reaktivproduktes dar, das als $CO_2$-Bildner ein verhältnismässig feinteiliges Calciumcarbonat enthält, dargestellt durch die Teilchen 23. Jeder Kern ist zu mindestens 80 % seiner Oberfläche von den Teilchen 23 bedeckt.

Zwischen den Kernen und den Carbonatteilchen 23 sind die strukturell vereinfachten und anteilsmässig übertriebenen Bindemittelschichten 22 dargestellt, wie sie in situ durch Anreaktion der Säure mit dem Calciumcarbonat in Gegenwart gering bemessener Mengen Wasser nach dem erfindungsgemässen Verfahren in reproduzierbarer Weise gebildet werden können, z. B. in Anteilen von 1 bis 5 % des Gewichtes der Säure- und Carbonatteilchen.

Es versteht sich, dass die Kerne 21 normalerweise nicht kugelförmig, sondern allgemein körnig sind, wie dies für mehr oder weniger kristalline Substanzen typisch ist. Im übrigen können die sauren Kerne unterschiedliche Grössen haben, solange sie im Verhältnis zu den Carbonatteilchen relativ grob sind.

In Fig. 3 ist schematisch und stark vergrössert ein Teil eines erfindungsgemäss mit Magnesiumoxid und Kaliumcarbonat behandelten Zitronensäurekristalls 21' dargestellt. In einem ersten Zyklus ist dort eine Schicht 22' aus Magnesiumoxid- und Zitronensäurepulver aufgetragen, wobei die Magnesiumoxidteilchen 23' von einer (nicht dargestellten) Magnesiumcitratschicht umgeben sind. In einer zweiten Zyklus wurde eine Schicht 22" aus denselben Bestandteilen aufgetragen. In einem dritten Zyklus schliesslich wurden Kaliumcarbonat-Kristalle 25 aufgetragen, wobei sich an der Berührungszone eine Kaliumcitratschicht 24 gebildet hat. Es ist wohlverstanden, dass die hier als Linien gezogenen Grenzen zwischen den einzelnen Teilchen und Schichten nicht als solche bestehen, sondern zumindest teilweise durch Diffusion verwischt sind.

In analoger Weise kann ein erfindungsgemässes Reaktivprodukt so aufgebaut sein, dass die Teilchen 23' eine toxikologisch zulässige, wasserlösliche Fluorverbindung, wie z. B. Alkalifluorophosphat, enthalten, wie später anhand des Beispiels 3 noch erläutert wird.

Im übrigen soll die Erfindung durch die nachfolgenden Beispiele weiter erläutert, nicht aber beschränkt werden.

## Beispiel 1:

In eine 720 Liter grosse Vakuummischtrommel mit einem nutzbaren Volumen von 300 Liter werden 200 kg einer Pulvermischung eingesetzt, die aus 150 kg Naproxen, 22 kg Füllstoff, 20 kg wasser und 8 kg Polyvinylpyrrolidon besteht.

Zunächst wird das Vakuumventil geöffnet und während etwa 50 Sekunden ein Druck von 200 mbar erzielt. Das Vakuumventil wird anschliessend geschlossen und über den Heisslufterzeuger während 30 bis 120 sekunden 130 bis 140 Grad heisse Luft eingedrückt, bis in der Behandlungstrommel ein Druck von etwa 1 bar herrscht. Anschliessend wird wieder für 50 Sekunden Vakuum angelegt und die Behandlung zyklisch wiederholt. Während der Heissluft-Druckphase erfolgt dabei die Migration der Feuchtigkeit aus dem Korninneren nach aussen, und die Abgabe der bereits aussen befindlichen Feuchtigkeit an die Heissluft. Es findet auch eine gewisse Konvektion statt. Während der Vakuumphase erfolgt die Abfuhr der mit Dampf beladenen Luft. Innerhalb von 20 Minuten wird die Gesamtmenge der Pulverschüttung auf eine Restfeuchtigkeit von 0,1 bis 0,2 % getrocknet.

## Beispiel 2:

Dieses Beispiel erläutert die Herstellung eines für Brause-Zubereitungen geeigneten Reaktivproduktes.

43 kg Zitronensäure mit Korngrössen zwischen 0,4 und 0,6 mm werden mit 22 kg Zitronensäure der Korngrösse 0,1 mm vermischt und in einem Vakuum-Mischkessel (Prozesskammer 14) mit einer Manteltemperatur von 65 Grad C auf 45 Grad C erhitzt. Dann werden 20 kg mikronisiertes Calciumcarbonat eingebracht; nach dem Evakuieren zur Entfernung allfälliger Restfeuchtigkeit wird das Gut wiederum auf 45 Grad C anwärmen gelassen.

Nun wird am Vakuum-Mischkessel ein Konstantvakuum von 600 bis 900 mbar) angelegt und das Heizgerät (Wärmetauscher 13) für die Lufterhitzung auf 120 Grad C (Gasaustrittstemperatur) eingestellt.

Die Pumpen 11, 16 und die Ventile 142, 152 sind so eingestellt, dass bei 700 mbar etwa 600 bis 800 Liter Heissluft pro Minute in die Prozesskammer 14 einströmen. Man lässt nun innerhalb von 2 min eine Menge von 500 ml Wasser aus dem Behälter 12 in den Wärmetauscher 13 strömen, wobei sich ein Verhältnis von 1 Volumenteil Wasserdampf auf 2 bis 3 Volumenteile Heissluft einstellt. Da der Siedepunkt des Wassers bei 900 mbar etwa 96 Grad C beträgt, kondensiert der Wasserdampf auf dem Prozessgut von etwa 45 Grad C. Unter dreidimensionalem Mischen während des Einsaugens kondensiert der Wasserdampf im Prozessgut in Form feinster Tröpfchen. Gleichzeitig beginnt die Entwicklung von $CO_2$, das über die Vakuumpumpe 16 abgezogen wird.

Nach Einbringen der genannten Wassermenge wird die Reaktion eine Minute laufen gelassen und dann volles Vakuum angelegt. Bei ausreichend dimensionierter Vakuumpumpe (100-200 $m^3$ pro Stunde) kann die Wasserentfernung bei etwa 20 mbar innerhalb von 2 bis 5 min erzielt werden.

Hierbei ergibt sich folgende Wärmebilanz:
Die kondensierte Wassermenge bewirkt eine Kalorienzufuhr von etwa 10.500 Kilojoule (2500 Kalorien). Dem Prozessgut kann man näherungsweise eine spezifische Wärme von 0,84 Kilojoule (0,2 Kalorien) pro Kilogramm zuschreiben. Da die vorgelegten 85 kg Prozessgut dementsprechend nur 71,2 Kilojoule (17 Kalorien) benötigen, damit sie um 1 Grad C erwärmt werden, hat sich die Temperatur des Prozessgutes bei diese Vorgang um etwa 15 Grad C (von 45 Grad C auf 60 Grad C) erwärmt.

Durch das Anlegen des Vakuums bzw. die dadurch bedingte Verdampfung wird diese Energie wieder verbraucht, so dass nach der Wasserentfernung des ersten Zyklus die ursprüngliche Temperatur des Gutes von 45 Grad C wieder erreicht werden sollte. Da aber die Manteltemperatur der Prozesskammer 65 Grad C beträgt, stellt sich nach dem ersten Zyklus eine Prozessguttemperatur von etwa 50 Grad C ein, da durch die Konvektion des einströmenden Wasserdampf/Heissluft-Gemisches an der Kammerwand der Wärmeübergang etwas verstärkt wird. Die Reaktionstemperatur mit Calciumcarbonat ist bei einer Prozessguttemperatur von 50 Grad C noch vernachlässigbar gering.

Wenn man diesen Zyklus wiederholt, stellt sich nach der Wasserentfernung eine Prozessguttemperatur von etwa 55 Grad C ein, was bereits zur Bildung eines stabilen trockenen Endproduktes genügt, wenn die Restfeuchtigkeit mit etwa 10 mbar Unterdruck entfernt wird. Wiederholt man den Zyklus ein drittes Mal, dann ist die Stabilität des Endproduktes noch höher, die Brausewirkung aber schon etwas verringert, da bereits etwa 20 % des ursprünglich vorgelegten Calciumcarbonates in der Zwischenschicht zu Calciumcitrat umgesetzt sind.

Durch Veränderung der eingesetzten Menge Wasserdampf, der Temperaturen und des Vakuums kann man die Reaktivität bzw. Brausetemperatur des Endproduktes wunschgemäss steuern. Das einmal festgelegte Verfahren kann z. B. wie folgt programmässig mit der beschriebenen Vorrichtung gesteuert werden:

Das beispielsweise über ein Manometer gesteuerte Regelventil 142, das in der Prozesskammer 14 einen Druck von beispielsweise 700 mbar hält, wird zum gewählten Programmzeitpunkt geschlossen, so dass die Pumpe 16 die Prozesskammer 14 auf volles Vakuum bringt.

Wird das Ventil 142 wieder geöffnet, dann gelangt bei entsprechender Einstellung in einer gewählten Zeiteinheit von beispielsweise 2 min eine Menge von beispielsweise 500 ml Wasser in die Prozesskammer 14. Nach dieser Zeitspanne wird das Ventil 142 geschlossen und z. B. nach weiteren 60 sec. das Ventil 152 zum Erzielen des vollen Vakuums geöffnet. Hat das Vakuum in der Prozesskammer einen Endwert von z. B. 20 mbar erreicht, kann der Zyklus wiederholt werden.

Nach zwei oder drei Zyklen kann die Prozesskammer zur abschliessenden Trocknung des Prozessgutes etwa 15 min auf Werte von unter 10 mbar gehalten werden, wodurch auch das reaktiv gebildete Calciumcitrat entwässert wird und auf diese Weise stabilisierend für das Produkt wirkt.

**Beispiel 3:**

33 kg kristallisierte Zitronensäure der Kristallgrösse 0,3 bis 0,6 mm, 10 kg Zitronensäure pulverisiert, 25 kg mikronisiertes Calciumcarbonat, sowie 3 kg Natriumfluorophosphat und 200 g Lebensmittelfarbstoff werden in die Reaktionskammer 14 eingebracht und darin auf 45 Grad C erwärmt.

Nun wird unter den Bedingungen von Beispiel 2 der Zyklus insgesamt dreimal durchgeführt. Nach der abschliessenden Trocknung ist sowohl der Farbstoff als auch das Natriumfluorophosphat durch dessen leichte Wasserlöslichkeit unter Einwirkung des kondensierenden Dampfes sehr gleichmässig verteilt; sowohl bei Betrachtung der einzelnen Teilchen unter dem Mikroskop als auch bei der Analyse sind keine signifikanten Verteilungsdifferenzen feststellbar.

**Beispiel 4:**

90 Teile Rohrzucker und 10 Teile Vitamin C werden in einem Vakuummischkessel auf etwa 40 Grad C gebracht; man schaltet sodann ein Konstantvakuum von 600 - 900 mbar ein. Anschliessend lässt man innerhalb einer Minute eine Wassermenge von einem Teil in einer Luftmenge von etwa 20 Teilen durch die Mischung strömen, wobei man bei hoher Rührgeschwindigkeit dreidimensional (schwingend) mischt. Der Vorgang dauert etwa 60 Sekunden. Sodann wird das Ventil zur Luftzufuhr geschlossen und das niedrige Konstantvakuum über eine Ventilsteuerung durch die volle Pumpenleistung ersetzt. Der Trocknungsvorgang dauert etwa 60 Sekunden. Nach diesen 60 Sekunden ist ein Endwert von etwa 30 mbar erreicht.

In diesem Moment wird das Ventil zur Luft-Dampf-Zufuhr geöffnet und wiederum auf das vorherige Konstantvakuum umgeschaltet. Diese Schritte können 5 - 10 Mal wiederholt werden, wobei ein gesamter Zeitaufwand von maximal 30 Minuten nötig ist. Das resultierende Granulat ist absolut gleichförmig, selbst zugefügte Farbstoffe wären absolut gleichmässig verteilt, und das Produkt kann über ein rotierendes Sieb vollkommen staubfrei zur Weiterverarbeitung oder Verpackung ausgetragen werden.

**Beispiel 5:**

95 Teile Lactose werden mit 4,5 Teilen Polyvinylpyrrolidon und 0,5 Teilen eines hochwirksamen Hormons vermischt und auf 40 Grad C erhitzt. Man legt dann ein Konstantvakuum von etwa 800 mbar an.

In diesem Falle lässt man als Agglomerationsmittel eine Lösung von Aceton in Luft einströmen. Diese Lösung kann aus 1 Teil Aceton in 5 - 10 Teilen Luft bestehen. Verwendet man hier beispielsweise Lactose mit einer Korngrösse von 0,2 mm, dann erhält man nach einer Behandlung von 5 Stufen eine totale Verklammerung des Hormons mit der Lactose, wobei bei den einzelnen Teilchen auch hier selbst bei Anwendung genauester Analytik keine Verteilungsunterschiede festgestellt werden können.

Besonders interessant wird das Verfahren aber dann, wenn man gleichzeitig so schwierig zu verteilende Stoffe wie Kolloide oder Pseudokolloide, Polymetacrylsäureester, Schellack, Wachse oder ähnliches mehr vorlegt.

Wiederholt man das Einströmen des Luft-Lösungsmittel-Gemisches in mehreren, gegebenenfalls auch 20 oder sogar 30 Stufen, dann erhält man jede gewünschte Art von verzögerter Wirkstoffreigabe in der einfachst denkbaren Form und unter Einsatz leicht durchzuführender Automatisationsmassnahmen.

**Beispiel 6:**

50 kg kristallisierte Zitronensäure der Kristallgrösse 0,3 - 0,6 mm, 15 kg Magnesiumoxidpulver und 15 kg Zitronensäure pulverisiert, werden in den Reaktionskessel eingebracht und auf 50 Grad C erwärmt. Man stellt sodann auf ein Konstantvakuum von 700 mbar ein und lässt innerhalb von 2 Min. ein Dampf/Luftgemisch (1 Vol.teil Wasserdampf auf 20 Vol.teile Luft) mit einem Druck von 500 mbar durch die Mischung strömen, wobei mit 10 U/min dreidimensional gemischt wird. Anschliessend wird die Luftzufuhr geschlosssen und mit voller Pumpenleistung die Trocknung bis 20 mbar ca. 2 Min. durchgeführt. Dabei klammert sich das Magnesiumoxid, unterstützt durch das Zitronensäurepulver, an der Oberfläche der Zitronensäurekristalle unter Bildung einer Magnesiumcitratbindeschicht an. Dieses Vorgehen wird zyklisch viermal wiederholt, wobei beim vierten Mal 15 kg Kaliumcarbonat wasserfrei (70 % unter 0,2 mm Korngrösse) zugeführt werden, das nunmehr mit den noch freien Oberflächen der Zitronensäure teilweise reagiert und seinerseits dort unter Bildung einer Kaliumcitratschichte verankert wird. Anschliessend wird auf 10 mbar unter rühren mit 2 U/min endgetrocknet. Zum Abschluss werden natürliches Aroma, Süss- und Füllstoffe zugefügt.

**Beispiel 7:**

40 kg Zitronensäure der Korngrösse 0,4 - 0,6 mm, 2,4 kg Süss-stoff und 18 kg Eisen-2-gluconat werden in einen Vakuumgranulator eingebracht der eine Manteltemperatur von 65 Grad C aufweist. Die Masse wird auf 50 Grad C erhitzt.

Am Vakuummischkessel wird ein Konstantvakuum von 600 mbar eingestellt. Das Heizgerät für die Lufterhitzung wird auf 120 Grad C eingestellt; die Pumpen 11, 16 und die Ventile werden so geregelt, dass bei 600 mbar 500 l Heissluft pro Minute in die Prozesskammer einströmen. Man lässt nun innerhalb von 2 Min. eine Menge von 400 ml Wasser aus dem Behälter in den Wärmeaustauscher strömen und verdampfen. Mit dieser Dampf/Luft-Mischung wird durch das Kondensieren auf dem Prozessgut das Eisengluconat auf die Zitronensäure granuliert.

Nach Einbringen der Wassermenge wird volles Vakuum angelegt, auf 20 mbar unter gelegentlichem Rühren getrocknet und das Wasser in ca. 5 Min. entfernt. Sodann gibt man zum Prozessgut 10 kg Natriumbicarbonat und wiederholt den Vorgang in gleicher Weise, wobei eine Anreaktion zwischen der Zitronensäure und dem Natriumbicarbonat erfolgt.

Nach der Trocknung auf 20 mbar werden nochmals 10 kg Natriumbicarbonat zugesetzt, und die zyklische Behandlung wird wiederholt. Das Produkt wird nun bei einer Temperatur von 50 Grad C auf 20 mbar endgetrocknet. Sodann werden 3 kg Aroma und 4 kg Füllstoffe zugesetzt, und das Granulat wird über die Siebmaschine ausgelassen.

**Patentansprüche**

1. Verfahren zur Behandlung von Prozessgut in einer geschlossenen Trommel, wobei intermittierend bis zum Erreichen eines vorbestimmten Behandlungsergebnisses

a. über oder durch die Schüttung des Gutes ein heisser Gasstrom geführt und

b. durch eine Vakuumpumpe wieder abgesaugt wird,

dadurch gekennzeichnet, dass der Heissgastrom vor seinem Eintritt in die Trommel unter Druck gesetzt und/oder mit einem dampfförmigen Behandlungsmittel beladen wird, wobei der Trommelmantel vorzugsweise beheizt ist.

2. Verfahren nach Anspruch 1, dadurch gekennzeichnet, dass das Heissgas eine solche Temperatur aufweist und mit einer solchen Dampfmenge beladen ist, dass der Dampf auf dem Gut nicht kondensiert.

3. Verfahren nach Anspruch 1 oder 2, dadurch gekennzeichnet, dass - in einem Zeitpunkt des Absaugens gemessen - die Druckdifferenz zwischen dem Eintritt des Heissgases und seinem Austritt mindestens 600, vorzugsweise mindestens 800 mbar beträgt.

4. Verfahren nach einem der vorhergehenden Ansprüche, dadurch gekennzeichnet, dass bei einer Masse von 10 kg alle 10 bis 120 Sekunden, vorzugsweise alle 20 bis 60 Sekunden das Gas während 5 bis 30, vorzugsweise während 15 bis 30 Sekunden abgesaugt wird.

5. Verfahren nach einem der vorhergehenden Ansprüche, dadurch gekennzeichnet, dass alle 60 bis 300, vorzugsweise alle 120 bis 180 Sekunden das Gut durch wenigstens eine Trommel- und/oder Rührerumdrehung bewegt wird.

6. Verfahren nach einem der vorhergehenden Ansprüche, dadurch gekennzeicnet, dass die Heissgaszufuhr während wenigstens eines Teiles der Absaugephase unterbrochen wird.

7. Verfahren nach einem der Ansprüche 1 bis 6, zur Herstellung eines Reaktivproduktes, das mindestens eine toxikologisch zulässige, wasserlösliche und unter Normalbedingungen feste Säure im verhältnismässig grobteiler Form, sowie mindestens einen feinteiligen Reaktionspartner enthält, dadurch gekennzeichnet, dass man in jedem Zyklus das als Behandlungsmittel verwendete Wasser in einer solchen Menge einsetzt und kondensiert, die jeweils für die Verankerung der zugesetzten Menge des feinteiligen Reaktionspartners gerade ausreicht.

8. Verfahren nach Anspruch 7, wobei der feinteilige Reaktionspartner Calciumcarbonat ist, dadurch gekennzeichnet, dass man auf 1 - 5, vorzugsweise auf 2 - 4 Volumsteile Heissluft jeweils 1 Vol.teil Wasserdampf zugibt.

9. Verfahren nach Anspruch 7, wobei einerseits ein Oxid, Hydroxid oder Salz, andererseits ein Erdalkalicarbonat oder -bicarbonat feinteilige Reaktionspartner sind, dadurch gekennzeichnet, dass zuerst in wenigstens einem Zyklus das Oxid, Hydroxid und/oder Salz, und anschliessend in wenigstens einem Zyklus das Alkalicarbonat und/oder Bicarbonat verankert wird.

10. Vorrichtung zur Durchführung des Verfahrens nach einem der vorhergehenden Ansprüche, bestehend aus einer - gegebenenfalls wenigstens einen Rührer (E) enthaltenden - dreh- und/oder schwenkbaren Trommel (14) mit einer Eintrittsöffnung (143) für das Heissgas in die Trommel und einer Pumpe (16) für die Evakuierung des Trommelinneren durch eine Absaugöffnung, wobei in der Leitung (151, 161) zur Pumpe (16) ein Ventil (152) angeordnet ist, dadurch gekennzeichnet, dass eine Vorrichtung (13) zur Erzeugung von Heissgas unter Druck und/oder eine Einrichtung (11, 12, 13) zur Erzeugung einer Mischung aus Heissgas und verdampftem Behandlungsmittel mit der Eintrittsöffnung (143) durch eine Leitung (141) verbunden sind, in der gegebenenfalls ein Ventil (142) angeordnet ist, sowie dass ein Mikroprozessor (17) für die intermittierende Betätigung des Ventils (145) - und gegebenenfalls des Ventils (142) - vorgesehen ist.

11. Vorrichtung nach Anspruch 10, dadurch gekennzeichnet, dass - auf Normaldruck bezogen - die Vakuumpumpe (16) die eineinhalb- bis vierfache, vorzugsweise die zwei- bis dreifache Literleistung des Heissgaserzeugers (11) aufweist.

12. Reaktivprodukt zur Verwendung als Brausezubereitung in Form von Granulat, mit einem Gehalt an einer kristallinen Säure, mindestens einem toxikologisch unbedenklichen Oxid, Hydroxid oder Salz, sowie einem Alkalicarbonat und/oder -bicarbonat, dadurch gekennzeichnet, dass die Säurekristalle wenigstens eine oberflächlich in einem ersten Bindemittel verankerte Schicht des Oxides, Hydroxides und/oder Salzes, und darüber wenigstens eine in einem zweiten Bindemittel verankerte Schicht des Alkalicarbonates und/oder -bicarbonates aufweisen, wobei das erste Bindemittel ein Reaktionsprodukt der Säure mit dem Oxid, Hydroxid und/oder Salz, das zweite Bindemittel ein Reaktionsprodukt der Säure mit dem Alkalicarbonat und/oder -bicarbonat ist.

13. Reaktivprodukt nach Anspruch 12, dadurch gekennzeichnet, dass als toxikologisch unbedenkliches Salz wenigstens eine der Verbindungen Alkalifluorophosphat, Eisengluconat, Zinkglycerophosphat und Calciumlactat, bzw. ein Magnesiumsalz vorliegt.

## Claims

1. Process for the treatment of processing material in a closed drum, in which, intermittently until a predetermined treatment result is achieved,

    a. a hot gas stream is fed over or through the bed of the material and

    b. is evacuated again by a vacuum pump,

characterized in that, before entering the drum, the hot gas stream is pressurized and/or is charged with a vapour treating agent, the drum shell preferably being heated.

2. Process according to Claim 1, characterized in that the temperature of the hot gas and the steam charge of the said gas are such that the steam does not condense on the material.

3. Process according to claim 1 or 2, characterized in that the pressure difference between the entrance of the hot gas and its exit is at least 600, preferably at least 800, mbar, measured at a time of evacuation.

4. Process according to any of the preceding Claims, characterized in that, with a mass of 10 kg, the gas is evacuated for 5 to 30, preferably 15 to 30, seconds every 10 to 120 seconds, preferably every 20 to 60 seconds.

5. Process according to any of the preceding Claims, characterized in that the material is moved through at least one drum and/or stirrer revolution every 60 to 300, preferably every 120 to 180, seconds.

6. Process according to any of the preceding Claims, characterized in that the hot gas feed is interrupted for at least part of the evacuation phase.

7. Process according to any of Claims 1 to 6, for the preparation of a reactive product which contains at least one toxicologically admissible, water-soluble acid in relatively coarse-particle form, which is solid under normal conditions, and

21         EP 0 258 258 B1         22

at least one finely divided reactant, characterized in that, in each cycle, the water used as treating agent is employed and condensed in an amount which is just sufficient for anchoring the added amount of finely divided reactant.

8. Process according to Claim 7, in which the finely divided reactant is calcium carbonate, characterized in that 1 part by volume of steam is added per 1 - 5, preferably 2 - 4, parts by volume of hot air.

9. Process according to Claim 7, in which on the one hand an oxide, hydroxide or salt and on the other hand an alkaline earth metal carbonate or bicarbonate are finely divided reactants, characterized in that the oxide, hydroxide and/or salt is first anchored in at least one cycle, and then the alkali metal carbonate and/or bicarbonate is anchored in at least one cycle.

10. Device for carrying out the process according to any of the preceding Claims, consisting of a rotatable and/or pivoted drum (14) which may contain at least one stirrer (E) and has an inlet opening (143) for the hot gas into the drum and a pump (16) for evacuating the interior of the drum through an evacuation opening, a valve (152) being arranged in the line (151, 161) to the pump (16), characterized in that a device (13) for producing hot gas under pressure and/or a means (11, 12, 13) for producing a mixture of hot gas and vaporized treating agent are connected to the inlet opening (143) by a line (141) in which a valve (142) may be arranged, and a microprocessor (17) for the intermittent operation of the valve (152) - and, if appropriate, the valve (142) - is provided.

11. Device according to Claim 10, characterized in that - based on atmospheric pressure - the vacuum pump (16) has one and a half to four times, preferably twice to three times, the litre capacity of the hot gas generator (11).

12. Reactive product for use as an effervescent formulation in the form of granules, containing at least one toxicologically safe oxide, hydroxide or salt and an alkali metal carbonate and/or bicarbonate, characterized in that the acid crystals have at least one layer of oxide, hydroxide and/or salt, which layer is superficially anchored in a first binder, and, on top of this, at least one layer of the alkali metal carbonate and/or bicarbonate, which layer is anchored in a second binder, the first binder being a reaction product of the acid with the oxide, hydroxide and/or salt, and the second binder being a reaction product of the acid with the alkali metal carbonate and/or bicarbonate.

13. Reactive product according to Claim 12, characterized in that at least one of the compounds from amongst alkali metal fluorophosphate, iron gluconate, zinc glycerophosphate and calcium lactate, or a magnesium salt, is present as the toxicologically safe salt.

## Revendications

1. Procédé de traitement de produit en cours d'élaboration dans un tambour fermé, dans lequel, de manière intermittente, jusqu'à l'obtention d'un résultat de traitement prédéterminé,

a) un courant de gaz chaud passe au-dessus ou au travers de la charge de produit à l'état de vrac, et

b) est réaspiré par une pompe à vide,

caractérisé par le fait qu'avant son entrée dans le tambour, le courant de gaz chaud est mis sous pression et/ou est chargé d'un agent de traitement sous forme de vapeur, la paroi du tambour étant de préférence chauffée.

2. Procédé selon revendication 1, caractérisé par le fait que la température du gaz chaud et la quantité de vapeur dont ce gaz est chargé sont telles que la vapeur ne se condense pas sur le produit.

3. Procédé selon revendication 1 ou 2, caractérisé par le fait que, mesurée à l'instant de l'aspiration, la différence de pression entre l'entrée et la sortie du gaz chaud est d'au moins 600 mbar, de préférence d'au moins 800 mbar.

4. Procédé selon l'une des revendications précédentes, caractérisé par le fait que, pour une masse de 10 kg, le gaz est aspiré toutes les 10 à 120 secondes, de préférence toutes les 20 à 60 secondes, pendat 5 à 30 secondes, de préférence pendant 15 à 30 secondes.

5. Procédé selon l'une des revendications précédentes, caractérisé par le fait que toutes les 60 à 300 secondes, de préférence toutes les 120 à 180 secondes, le produit est déplacé par au moins une rotation du tambour et/ou de l'agitateur.

6. Procédé selon l'une des revendications précédentes, caractérisé par le fait que l'amenée de gaz chaud est interrompue pendant au moins une partie de la phase d'aspiration.

7. Procédé selon l'une des revendications 1 à 6, pour préparer un produit réactif contenant au moins un acide toxicologiquement admissible, soluble dans l'eau et solide dans les conditions normales, sous forme de grains relativement gros, ainsi qu'au moins un partenaire réactionnel en grains fins, caractérisé par le fait que, lors de chaque cycle, l'eau utilisée comme agent de traitement est apportée et condensée en quantité à chaque fois suffisante pour la fixation de la quantité ajoutée du partenaire réactionnel en grains fins.

8. Procédé selon revendication 7, dans lequel le partenaire réactionnel en grains fins est le carbonate de calcium, caractérisé par le fait que l'on ajoute une part d'eau en volume à 1 - 5 parts, de préférence 2 - 4 parts, en volume d'air chaud.

9. Procédé selon revendication 7, dans lequel un oxyde, hydroxyde ou sel, d'une part, un carbonate alcalino-terreux ou un bicarbonate alcalino-terreux, d'autre part sont des partenaires réactionnels en grains fins, caractérisé par la fixation, en premier, en au moins un cycle, de l'oxyde, hydroxyde et/ou sel, suivie par la fixation, en au moins un cycle, du carbonate alcalin et/ou du bicarbonate alcalin.

10. Dispositif de mise en oeuvre du procédé selon l'une des revendications précédentes, constitué par un tambour (14) rotatif et/ou pivotant, contenant éventuellement au moins un agitateur (E), ce tambour étant muni d'une ouverture (143) pour l'entrée du gaz chaud, et une pompe (16) étant prévue pour vider l'intérieur du tambour par une ouverture d'aspiration, une vanne (152) étant montée sur la conduite (151, 161) allant à la pompe (16), caractérisé par le fait qu'un dispositif (13) de production de gaz chaud sous pression et/ou un dispositif (11, 12, 13) de production d'un mélange de gaz chaud et d'agent de traitement vaporisé sont reliés à l'ouverture d'entrée (143) par une conduite (141) sur laquelle est éventuellement montée une vanne (142), et par le fait qu'un microprocesseur (17) est prévu pour l'actionnement intermittent de la vanne (152), et éventuellement de la vanne (142).

11. Dispositif selon revendication 10, caractérisé par le fait que, par rapport à la pression normale, la pompe à vide (16) présente un débit volumique de une fois et demie à quatre fois, de préférence de deux à trois fois celui du générateur de gaz chaud (11).

12. Produit réactif pour utilisation en tant que préparation pour arrosage, sous forme de granulé contenant au moins un oxyde, hydroxyde ou sel toxicologiquement inoffensif, ainsi qu'un carbonate alcalin et/ou un bicarbonate alcalin, caractérisé par le fait que les cristaux d'acide présentent au moins une couche de l'oxyde, hydroxyde et/ou sel fixée superficiellement dans un premier liant et, par-dessus, au moins une couche du carbonate alcalin et/ou du bicarbonate alcalin fixée dans un second liant, le premier liant étant un produit de réaction de l'acide avec l'oxyde, l'hydroxyde et/ou le sel, le second liant étant un produit de réaction de l'acide avec le carbonate alcalin et/ou le bicarbonate alcalin.

13. Produit réactif selon revendication 12, caractérisé par le fait que le sel toxicologiquement inoffensif est au moins l'un des composés fluorophospate alcalin, gluconate de fer, glycérophosphate de zinc et lactate de calcium, ou un sel de magnésium.

FIG. 1

Fig. 2

Fig. 3